# EUROPÄISCHE PATENTSCHRIFT

(11) **EP 1 191 014 B1**
(45) Veröffentlichungstag und Bekanntmachung des Hinweises auf die Patenterteilung: **04.08.2004**
(21) Anmeldenummer: 01119685.4
(22) Anmeldetag: 23.08.2001
(51) Int. Cl.: C07C 201/08, C07B 43/02

(54) **Verfahren zur Nitrierung aromatischer Kohlenwasserstoffe**
Process for the nitration of aromatic hydrocarbons
Procédé de nitration d' hydrocarbures aromatiques

(30) Priorität: 22.09.2000 DE 10047163
(43) Veröffentlichungstag der Anmeldung: 27.03.2002
(73) Patentinhaber: BASF AKTIENGESELLSCHAFT, 67056 Ludwigshafen (DE)
(72) Erfinder: Eiermann, Matthias, Dr., 67117 Limburgerhof (DE); Ebel, Klaus, Dr., 68623 Lampertheim (DE)
(74) Vertreter: Isenbruck, Günter, Dr.

(56) Entgegenhaltungen:
- EP-A- 0 092 372
- EP-A- 0 169 441
- EP-A- 0 182 771
- WO-A-99/42433
- GB-A- 131 982
- US-A- 4 426 543
- US-A- 4 766 257

## Beschreibung

Die Erfindung betrifft ein Verfahren zur Nitrierung aromatischer Kohlenwasserstoffe.

Aromatische Nitroverbindungen sind wichtige Zwischenprodukte in der organischen Synthese und nehmen in der chemischen Industrie eine bedeutende Stellung ein. Diese werden beispielsweise zu Aminen reduziert und weiter zu Isocyanaten umgesetzt. So ist Nitrobenzol Ausgangsstoff für die Herstellung von Anilin, aus dem Methylendiphenyldiisocyanat (MDI), ein wichtiger Baustein für die Herstellung von Polyurethanschäumen, erhalten wird. Aromatische Nitroverbindungen sind ferner Grundlage für die Herstellung von Pflanzenschutz- und Pharmawirkstoffen, Farbstoffen und Sprengstoffen.

Aromatische Nitroverbindungen werden großtechnisch aus den entsprechenden Aromaten durch Nitrierung mit der sogenannten Nitriersäure, einem Gemisch aus konzentrierter Salpetersäure und Schwefelsäure, erhalten. Das Verfahren ist seit dem 19. Jahrhundert bekannt. Je nach Konzentration, Substitutionsmuster am Aromaten und Reaktionsbedingungen werden eine oder mehrere Nitrogruppen am Aromaten eingeführt. Nachteilig ist der Anfall von mit organischen Verbindungen belasteter Nitriersäure, die aufwendig entsorgt oder regeneriert werden muß. Es besteht daher das Bedürfnis, kostengünstige Alternativen zu diesem Verfahren zu finden, die sich großtechnisch umsetzen lassen.

In WO 99/42433 ist ein Verfahren zur Nitrierung aromatischer Verbindungen offenbart, bei dem die aromatische Verbindung in einem Autoklaven mit Stickstoffdioxid und Sauerstoff unter Druck in Gegenwart eines suspendierten Oxidkatalysators umgesetzt wird. Dabei wird mit einem Sauerstoff-Partialdruck von 3 bis 8 bar gearbeitet. Nachteilig sind die geringen Umsätze in diesem Verfahren.

GB 131,982 offenbart ein Verfahren zur Nitrierung von Benzol oder Anisol, bei dem Sauerstoff oder Luft unter Druck in ein Gemisch der zu nitrierenden Verbindung mit flüssigem N₂O₄ in Gegenwart von Wasserspuren eingeleitet wird. In Gegenwart von Sauerstoff und den Wasserspuren bildet sich aus N₂O₄ HNO₃, welches als nitrierendes Agens wirkt. Die Anwesenheit heterogenoxidischer Katalysatoren wird nicht erwähnt.

Aufgabe der Erfindung ist es, ein alternatives Verfahren zur Nitrierung aromatischer Kohlenwasserstoffe bereitzustellen, das hohe Umsätze ermöglicht.

Gelöst wird die Aufgabe durch ein Verfahren zur Nitrierung eines aromatischen Kohlenwasserstoffs, bei dem der aromatische Kohlenwasserstoff in flüssiger Phase mit einem Stickstoffoxid, ausgewählt aus NO, N₂O₃, NO₂ und N₂O₄, und einem sauerstoffhaltigen Gasstrom in Gegenwart eines heterogenen oxidischen Katalysators umgesetzt wird, dadurch gekennzeichnet, daß zu Beginn der Umsetzung in Gegenwart von mindestens 0,1 mol.-% Wasser, bezogen auf den zu nitrierenden aromatischen Kohlenwasserstoff, gearbeitet wird.

Es wurde gefunden, daß der Umsatz der Nitrierung mit Stickstoffoxiden und Sauerstoff deutlich gesteigert wird, wenn die Nitrierung in Gegenwart geringer Wassermengen gestartet wird.

Bevorzugt wird mit einer Wassermenge zu Beginn der Nitrierung von mindestens 0,5 mol.-%, besonders bevorzugt mindestens 1 mol.-%, bezogen auf die zu nitrierenden aromatischen Kohlenwasserstoffe, gearbeitet.

Das Wasser kann in flüssiger Phase oder als Dampf eingesetzt werden. Beispielsweise kann das Wasser im Gemisch mit den zu nitrierenden aromatischen Kohlenwasserstoffen in flüssiger Phase der Umsetzung zugeführt oder von diesen getrennt als Flüssigkeit oder als Wasserdampf zugeführt werden. Das Wasser kann auch in Form einer wasserhaltigen Flüssigkeit, vorzugsweise Salpetersäure, eingesetzt werden.

Das Stickstoffoxid kann in flüssiger Phase oder als stickstoffoxidhaltiger Gasstrom eingesetzt werden. In einer Ausführungsform der Erfindung wird flüssiges Distickstofftetroxid eingesetzt, das im Gemisch mit den zu nitrierenden Aromaten der Umsetzung zugeführt werden kann. In einer bevorzugten Ausführungsform wird ein Stickstoffmonoxid und/oder Stickstoffdioxid enthaltender Gasstrom eingesetzt. Dieser kann Inertgase wie Stickstoff enthalten. Bevorzugt wird reines Stickstoffdioxid eingesetzt.

Sauerstoff kann als Reinsauerstoff, mit Inertgasen verdünnt oder in Form von Luft eingesetzt werden. Bevorzugt wird Reinsauerstoff eingesetzt.

Der Einsatz der reinen Reaktionsgase ist im Interesse einer hohen Reaktionsgeschwindigkeit und geringer Abgasmengen bevorzugt.

Es können auch technische Gemische von Stickstoffdioxid und Sauerstoff oder Luft, wie sie beispielsweise bei der Salpetersäureherstellung erzeugt werden, eingesetzt werden.

Geeignete aromatische Kohlenwasserstoffe sind im allgemeinen monocyclische oder polycyclische aromatische Kohlenwasserstoffe, die beispielsweise mit Nitro-, Nitroso-, Halogen-, Hydroxy-, Alkoxy-, Aryloxy-, Carboxy-, Alkylcarbonyloxy-, Arylcarbonyloxy-, Acylamino-, Alkylsulfonyl-, Arylsulfonyl- Alkylsulfoxy-, Arylsulfoxy- Sulfonsäure-, Cyano- und/oder Alkylgruppen (Alkyl ist vorstehend im allgemeinen C₁-C₁₈-Alkyl), bevorzugt mit Nitro-, Halogen-, Cyano- oder/oder Alkylgruppen, ein oder mehrfach substituiert sein können. Ungeeignet im Sinne einer selektiven Nitrierung des aromatischen Ringes sind Amino-, Alkylamino- oder Dialkylaminogruppen, niedervalenten Schwefel oder Phosphor enthaltende Substituenten und andere leicht oxidierbare oder nitrierbare Gruppen. Bevorzugte aromatische Kohlenwasserstoffe sind unsubstituiertes oder substituiertes Benzol, Naphthalin, Biphenyl, Anthracen und Phenanthren.

Es können selbstverständlich auch Gemische aromatischer Kohlenwasserstoffe eingesetzt werden.

Der aromatische Kohlenwasserstoff kann in Reinsubstanz oder in einem Lösungsmittel gelöst nitriert werden. Geeignete Lösungsmittel sind beispielsweise Halogenkohlenwasserstoffe wie Dichlormethan, Chloroform, Tetrachlorkohlenstoff und Bromoform, Acetonitril oder reaktionsträge elektronenarme aromatische Kohlenwasserstoffe wie Dinitrobenzql oder Benzonitril. Als Lösungsmittel kann femer Salpetersäure eingesetzt werden. Es können auch Gemische verschiedener Lösungsmittel eingesetzt werden. Bevorzugt wird ohne Lösungsmittel oder in dem Nitrierungsprodukt als Lösungsmittel gearbeitet.

Als oxidischer Katalysator geeignet sind feste, im Reaktionsmedium unlösliche Sauerstoffverbindungen von einem oder mehreren Elementen, ausgewählt aus den Elementen der dritten und vierten Hauptgruppe und der dritten und vierten Nebengruppe (Gruppen IIIa, IVa, IIIb oder IVb) des Periodensystems. Insbesondere geeignet sind Sauerstoffverbindungen von Silizium, Aluminium, Zirkon, Bor und Titan, die in ihrer aktiven Form saure Zentren an der Katalysatoroberfläche aufweisen. Es können auch Silikate, Borosilikate und Alumosilikate von Alkali-, Erdalkalimetallen und Metallen der vierten Nebengruppe wie Magnesiumsilikat oder Titansilikat, ferner Zeolithe wie FSM-5, NaY, Mordenit oder Faujasit eingesetzt werden. Geeignet sind auch natürlich vorkommende Materialien wie Kaolinit, Fullererden oder Diatomeenerde.

Bevorzugt beträgt die BET-Oberfläche der eingesetzten Katalysatoren mindestens 50 m²/g. Geringere spezifische Oberflächen führen zu deutlich verminderter Katalysatoraktivität. Die Katalysatoren können beispielsweise durch Fällung geeigneter Vorläuferverbindungen, Trocknen und Calcinieren hergestellt werden.

Besonders bevorzugte Katalysatoren sind Siliziumdioxid, mit Lanthanoxid dotiertes Siliziumdioxid enthaltend bis zu 10 Gew.-% La₂O₃, Kaolinit, NaY-Zeolith, Titandioxid und Zirkondioxid.

Das erfindungsgemäße Verfahren kann diskontinuierlich oder kontinuierlich durchgeführt werden. Diskontinuierlich kann das Verfahren beispielsweise in einem Rührbehälter unter Druck mit in dem zu nitrierenden aromatischen Kohlenwasserstoff suspendiertem Katalysator durchgeführt werden. Das Stickstoffoxid kann als flüssiges Distickstofftetroxid zusammen mit dem aromatischen Kohlenwasserstoff vorgelegt werden oder aber mit dem sauerstoffhaltigen Gasstrom als gasförmiges Stickstoffoxid, bevorzugt Stickstoffdioxid, aufgepresst werden. Wasser oder die wasserhaltige Flüssigkeit können in flüssiger Phase vorgelegt oder als Dampf aufgepresst werden. Nachteilig an der diskontinuierlichen Betriebsweise ist jedoch der starke Katalysatorabrieb sowie die sich ständig ändernden Verfahrensparameter wie Druck, Temperatur und Zusammensetzung des Reaktionsgemischs. Die sich ändernden Verfahrensbedingungen erschweren einen sicheren Betrieb von diskontinuierlich arbeitenden Reaktoren, so daß diese häufig wegen der Gefahr der Ausbildung explosionsfähiger Gasgemische in die höchste Explosionsschutzklasse eingestuft werden müssen. Die diskontinuierliche Betriebsweise ist daher weniger bevorzugt.

Bevorzugt wird das erfindungsgemäße Verfahren kontinuierlich durchgeführt.

In einer Ausführungsform wird eine flüssige Mischung, enthaltend einen oder mehrere zu nitrierende aromatische Kohlenwasserstoffe und gegebenenfalls ein Lösungsmittel, zusammen mit dem das Stickstoffoxid und den Sauerstoff enthaltenden Gasstrom, Wasser oder Wasserdampf kontinuierlich über ein Katalysator-Festbett geleitet. Dabei wird die flüssige Phase im Gleichstrom oder im Gegenstrom zur Gasphase über das Katalysator-Festbett geführt, beispielsweise in Rieselbett- oder Sumpffahrweise. Geeignete Reaktoren sind beispielsweise Rohr- oder Rohrbündelreaktoren.

Es ist auch möglich, bei hinreichendem Dichteunterschied zwischen Katalysator und Reaktionsmedium und hinreichender Sedimentationsgeschwindigkeit den festen Katalysator in der aufsteigenden Flüssigphase eines Reaktionsrohres suspendiert zu halten und eine Katalysatorabtrennung über eine Sedimentationszone am Reaktorkopf oder eine Filtereinrichtung vorzunehmen.

Geeignet sind auch Reaktionskolonnen, in denen sich das Katalysatorbett in geeigneten Haltevorrichtungen, bei Bodenkolonnen beispielsweise auf den Kolonnenböden, befindet.
Es können auch kontinuierlich betriebene Kessel oder Kesselkaskaden, die mit Umwälzeinrichtungen wie Rührern oder Umwälzpumpen ausgestattet sind, eingesetzt werden.

Bei allen Verfahren kann die erforderliche Wassermenge in reiner Form oder in Form einer wasserhaltigen Flüssigkeit wie Salpetersäure zusammen mit dem Aromaten(gemisch) oder getrennt davon als Flüssigkeit, oder aber als Dampf zusammen mit oder getrennt von den übrigen Reaktionsgasen dem Reaktor zugeführt werden.

Bevorzugt wird mit einem Katalysator-Festbett in Rieselbett-Fahrweise gearbeitet.

Das erfindungsgemäße Verfahren wird in Gegenwart einer festen Katalysatorphase, einer flüssigen Eduktphase und einer zwei Reaktanten enthaltenden Gasphase durchgeführt. Als Kombination einer Nitrierungs- und einer Oxidationsreaktion ist die erfindungsgemäße Nitrierung insgesamt in besonderem Maße von den Reaktionsgeschwindigkeiten der Einzelschritte abhängig. Das Zusammenwirken von Absorptions- und Desorptionsvorgängen in Gas- und Flüssigphase und der einzelnen Reaktionsschritte läßt sich kinetisch nur schwierig beschreiben. Dennoch läßt sich das erfindungsgemäße Verfahren überraschenderweise an einem Katalysator-Festbett oder -Wirbelbett mit vom Rührkessel stark abweichendem Verweilzeit- und Durchmischungsverhalten durchführen.

Druck und Temperatur werden so gewählt, daß eine ausreichende Reaktionsgeschwindigkeit resultiert. Diese hängt auch von der Reaktivität der zu nitrierenden aromatischen Verbindung ab. Obwohl in der Regel schon bei Normaldruck mit Luft eine Umsetzung beobachtet wird, wird im allgemeinen mit einem Sauerstoffpartialdruck von mindestens 2 bar gearbeitet, um beispielsweise Verbindungen mittlerer Reaktivität wie Benzol oder Naphthalin zu nitrieren. Hohe Drücke bis 300 bar sind möglich, aber aus wirtschaftlichen Gründen weniger bevorzugt. Bevorzugt sind Sauerstoffpartialdrücke von 2 bis 25 bar bei Aromaten mittlerer und geringer Reaktivität.

Temperatur und Verweilzeit werden in Abhängigkeit von der Reaktivität gewählt. Aromaten mittlerer Reaktivität wie Benzol und Naphthalin können bereits bei Raumtemperatur nitriert werden, bevorzugt wird bei 40 bis 80°C mit Verweilzeiten von 10 min bis 5 Stunden gearbeitet. Aromaten geringer Reaktivität wie Nitrobenzol oder Benzonitril erfordern im allgemeinen Temperaturen von 60 bis 180°C und Verweilzeiten von 30 min bis 20 Stunden. Letzteres gilt auch für Mehrfachnitrierungen.

## Patentansprüche

1. Verfahren zur Nitrierung eines monocyclischen oder polycyclischen aromatischen Kohlenwasserstoffs, der mit Nitro-, Nitroso-, Halogen-, Hydroxy-, Alkoxy-, Aryloxy-, Carboxy-, Alkylcarbonyloxy-, Arylcarbonyloxy-, Acylamino-, Alkylsulfonyl-, Arylsulfonyl-, Alkylsulfoxy-, Arylsulfoxy-, Sulfonsäure-, Cyanound/oder C₁-C₁₈-Alkyl ein- oder mehrfach substituiert sein kann, bei dem der aromatische Kohlenwasserstoff in flüssiger Phase mit einem Stickstoffoxid, ausgewählt aus NO, N₂O₃, NO₂ und N₂O₄, und einem sauerstoffhaltigen Gasstrom in Gegenwart eines heterogenen oxidischen Katalysators umgesetzt wird, **dadurch gekennzeichnet, daß** zu Beginn der Umsetzung in Gegenwart von mindestens 0,1 mol.-% Wasser, bezogen auf den aromatischen Kohlenwasserstoff, gearbeitet wird.

2. Verfahren nach Anspruch 1, **dadurch gekennzeichnet, daß** Wasser in flüssiger Phase oder als Dampf eingesetzt wird.

3. Verfahren nach Anspruch 1 oder 2, **dadurch gekennzeichnet, daß** Wasser in Form einer wasserhaltigen Flüssigkeit eingesetzt wird.

4. Verfahren nach Anspruch 3, **dadurch gekennzeichnet, daß** Wasser in Form von wässriger Salpetersäure eingesetzt wird.

5. Verfahren nach einem der Ansprüche 1 bis 4, **dadurch gekennzeichnet, daß** Stickstoffoxid in flüssiger Phase oder als stickstoffoxidhaltiger Gasstrom eingesetzt wird.

6. Verfahren nach einem der Ansprüche 1 bis 5, **dadurch gekennzeichnet, daß** Sauerstoff als Reinsauerstoff oder in Form von Luft eingesetzt wird.

7. Verfahren nach einem der Ansprüche 1 bis 6, **dadurch gekennzeichnet, daß** der aromatische Kohlenwasserstoff in einem Lösungsmittel gelöst vorliegt.

8. Verfahren nach einem der Ansprüche 1 bis 7, **dadurch gekennzeichnet, daß** der Katalysator eine Sauerstoffverbindung von einem oder mehreren Elementen der Gruppen IIIa, IVa, IIIb oder IVb ist.

9. Verfahren nach einem der Ansprüche 1 bis 8, **dadurch gekennzeichnet, daß** der aromatische Kohlenwasserstoff ein unsubstituierter oder gegebenenfalls mit Nitro-, C₁-C₁₈-Alkyl-, Cyano- oder Halogensubstituenten ein oder mehrfach substituierter mono- oder polycyclischer aromatischer Kohlenwasserstoff, ausgewählt aus der Gruppe bestehend aus unsubstituiertem oder substituierten Benzolen, Naphthalin, Biphenyl, Anthracen oder Phenanthren, ist.

10. Verfahren nach einem der Ansprüche 1 bis 9, **dadurch gekennzeichnet, daß** das Verfahren kontinuierlich an einem Katalysator-Festbett in Sumpf- oder Rieselfahreweise durchgeführt wird.

## Claims

1. A process for nitrating a monocyclic or polycyclic aromatic hydrocarbon which may be monosubstituted or polysubstituted by nitro, nitroso, halogen, hydroxyl, alkoxy, aryloxy, carboxyl, alkylcarbonyloxy, arylcarbonyloxy, acylamino, alkylsulfonyl, arylsulfonyl, alkylsulfoxy, arylsulfoxy, sulfo, cyano and/or C₁-C₁₈-alkyl, in which the aromatic hydrocarbon in the liquid phase is reacted with an oxide of nitrogen selected from NO, N₂O₃, NO₂ and N₂O₄ and with an oxygen-containing gas stream in the presence of a heterogeneous oxidic catalyst, wherein at least 0.1 mol%, based on the aromatic hydrocarbon, of water is present at the beginning of the reaction.

2. A process as claimed in claim 1, wherein water is used in the liquid phase or in the form of steam.

3. A process as claimed in claim 1 or 2, wherein water is used in the form of a water-containing liquid.

4. A process as claimed in claim 3, wherein water is used in the form of aqueous nitric acid.

5. A process as claimed in any of claims 1 to 4, wherein an oxide of nitrogen in the liquid phase or in the form of a gas stream containing the oxide of nitrogen is used.

6. A process as claimed in any of claims 1 to 5, wherein oxygen in the form of pure oxygen or in the form of air is used.

7. A process as claimed in any of claims 1 to 6, wherein the aromatic hydrocarbon is initially taken in solution in a solvent.

8. A process as claimed in any of claims 1 to 7, wherein the catalyst is an oxygen compound of one or more elements of groups IIIa, IVa, IIIb or IVb.

9. A process as claimed in any of claims 1 to 8, wherein the aromatic hydrocarbon is a mono- or polycyclic aromatic hydrocarbon which is unsubstituted or monosubstituted or polysubstituted by nitro, C₁-C₁₈-alkyl, cyano or halogen substituents and is selected from the group consisting of unsubstituted or substituted benzenes, naphthalene, biphenyl, anthracene and phenanthrene.

10. A process as claimed in any of claims 1 to 9, wherein the process is carried out continuously over a fixed catalyst bed by the liquid-phase or trickle-bed procedure.

## Revendications

1. Procédé de nitration d'un hydrocarbure aromatique monocyclique ou polycyclique, qui peut être substitué une ou plusieurs fois par des radicaux nitro, nitroso, halogéno, hydroxy, alcoxy, aryloxy, carboxy, alkylcarbonyloxy, arylcarbonyloxy, acylamino, alkylsulfonyle, arylsulfonyle, alkylsulfoxy, arylsulfoxy, acide sulfonique, cyano et/ou alkyle en C₁ à C₁₈, dans lequel on fait réagir l'hydrocarbure aromatique, en phase liquide, avec un oxyde d'azote choisi parmi NO, N₂O₃, NO₂ et N₂O₄, et un courant de gaz contenant de l'oxygène, en présence d'un catalyseur oxydique hétérogène, **caractérisé en ce qu'**au début de la réaction, on travaille en présence d'au moins 0,1% molaire d'eau, par rapport à l'hydrocarbure aromatique.

2. Procédé selon la revendication 1, **caractérisé en ce que** l'eau est mise en oeuvre sous forme liquide ou sous forme de vapeur.

3. Procédé selon la revendication 1 ou 2, **caractérisé en ce que** l'eau est mise en oeuvre sous forme d'un liquide contenant de l'eau.

4. Procédé selon la revendication 3, **caractérisé en ce que** l'eau est mise en oeuvre sous forme d'acide nitrique aqueux.

5. Procédé selon l'une quelconque des revendications 1 à 4, **caractérisé en ce que** l'on met en oeuvre l'oxyde d'azote en phase liquide ou sous forme d'un courant de gaz contenant de l'oxyde d'azote.

6. Procédé selon l'une quelconque des revendications 1 à 5, **caractérisé en ce que** l'oxygène est mis en oeuvre en tant qu'oxygène pur ou sous forme d'un courant de gaz contenant de l'oxygène.

7. Procédé selon l'une quelconque des revendications 1 à 6, **caractérisé en ce que** l'hydrocarbure aromatique est dissous dans un solvant.

8. Procédé selon l'une quelconque des revendications 1 à 7, **caractérisé en ce que** le catalyseur est un composé oxygéné d'un ou plusieurs éléments des groupes IIIa, IVa, IIIb ou IVb.

9. Procédé selon l'une quelconque des revendications 1 à 8, **caractérisé en ce que** l'hydrocarbure aromatique est un hydrocarbure aromatique monocyclique ou polycyclique non substitué ou éventuellement substitué une ou plusieurs fois par des substituants nitro, alkyle en C₁ à C₁₈, cyano ou halogène, choisi dans le groupe formé par du benzène, naphtalène, biphényle, anthracène ou phénanthrène non substitués ou substitués.

10. Procédé selon l'une quelconque des revendications 1 à 9, **caractérisé en ce que** le procédé est entrepris en continu sur un catalyseur à lit fixe en mode de fond de colonne ou à écoulement.
